# EUROPEAN PATENT SPECIFICATION

(11) **EP 1 744 736 B1**
(45) Date of publication and mention of the grant of the patent: **18.11.2009**
(21) Application number: 05751168.5
(22) Date of filing: 12.05.2005
(51) Int. Cl.: A61K 31/047, A61K 31/22, A61P 27/02

(54) **METHOD OF TREATING DRY EYE DISORDERS AND UVEITIS**
METHODE ZUR BEHANDLUNG TROCKENER AUGEN UND UVEITIS
PROCEDE DE TRAITEMENT DES PROBLEMES DES YEUX SECS ET DE L"UVEITE

(30) Priority: 14.05.2004 US 571162 P
(43) Date of publication of application: 24.01.2007
(73) Proprietor: Alcon, Inc., 6331 Hünenberg (CH)
(72) Inventor: KLIMKO, Peter, G., Fort Worth, TX 76110 (US); HELLBERG, Mark, R., Arlington, TX 76017 (US); GAMACHE, Daniel, A., Arlington, TX 76017 (US)
(74) Representative: Best, Michael
(86) International application number: PCT/US2005/016646
(87) International publication number: WO 2005/112905

(56) References cited:
- US-B1- 6 645 978
- LEE T H ET AL: "INHIBITION OF LEUKOTRIENE B4-INDUCED NEUTROPHIL MIGRATION BY LIPOXIN A4: STRUCTURE-FUNCTION RELATIONSHIPS" BIOCHEMICAL AND BIOPHYSICAL RESEARCH COMMUNICATIONS, ACADEMIC PRESS INC. ORLANDO, FL, US, vol. 180, no. 3, 14 November 1991 (1991-11-14), pages 1416-1421, XP008042397 ISSN: 0006-291X cited in the application

## Description

The present invention is directed to the preparation of a medicament for the treatment of dry eye disorders. In particular, the present invention is directed toward the use of 5,6,7-trihydroxyheptanoic acid and its analogs according to formula (1) for the preparation of medicament for the treatment of dry eye and uveitis in mammal.

### Background of the invention

Dry eye, also known generically as *keratoconjunctivitis sicca,* is a common ophthalmological disorder affecting millions of Americans each year. The condition is particularly widespread among post-menopausal women due to hormonal changes following the cessation of fertility. Dry eye may afflict an individual with varying severity. In mild cases, a patient may experience burning, a feeling of dryness, and persistent irritation such as is often caused by small bodies lodging between the eye lid and the eye surface. In severe cases, vision may be substantially impaired. Other diseases, such as Sjogren's disease and *cicatricial pemphigoid* manifest dry eye complications.

Although it appears that dry eye may result from a number of unrelated pathogenic causes, all presentations of the complication share a common effect, that is the breakdown of the pre-ocular tear film, which results in dehydration of the exposed outer surface and many of the symptoms outlined above (Lemp, Report of the National Eye Institute/Industry Workshop on Clinical Trials in Dry Eyes, The CLAO Journal, volume 21, number 4, pages 221-231 (1995)).

Practitioners have taken several approaches to the treatment of dry eye. One common approach has been to supplement and stabilize the ocular tear film using so-called artificial tears instilled throughout the day. Other approaches include the use of ocular inserts that provide a tear substitute or stimulation of endogenous tear production.

Examples of the tear substitution approach include the use of buffered, isotonic saline solutions, aqueous solutions containing water soluble polymers that render the solutions more viscous and thus less easily shed by the eye. Tear reconstitution is also attempted by providing one or more components of the tear film such as phospholipids and oils. Phospholipid compositions have been shown to be useful in treating dry eye; see, e.g., McCulley and Shine, Tear film structure and dry eye, Contactologia, volume 20(4), pages 145-49 (1998); and Shine and McCulley, Keratoconjunctivitis sicca associated with meibomian secretion polar lipid abnormality, Archives of Ophthalmology, volume 116(7), pages 849-52 (1998). Examples of phospholipid compositions for the treatment of dry eye are disclosed in U.S. Patent Nos. 4,131,651 (Shah et al.), 4,370,325 (Packman), 4,409,205 (Shively), 4,744,980 and 4,883,658 (Holly), 4,914,088 (Glonek), 5,075,104 (Gressel et al.), 5,278,151 (Korb et al.), 5,294,607 (Glonek et al.), 5,371,108 (Korb et al.) and 5,578,586 (Glonek et al.). U.S. Patent No. 5,174,988 (Mautone et al.) discloses phospholipid drug delivery systems involving phospholipids, propellants and an active substance.

Another approach involves the provision of lubricating substances in lieu of artificial tears. For example, U.S. Patent No. 4,818,537 (Guo) discloses the use of a lubricating, liposome-based composition, and U.S. Patent No. 5,800,807 (Hu et al.) discloses compositions containing glycerin and propylene glycol for treating dry eye.

Although these approaches have met with some success, problems in the treatment of dry eye nevertheless remain. The use of tear substitutes, while temporarily effective, generally requires repeated application over the course of a patient's waking hours. It is not uncommon for a patient to have to apply artificial tear solution ten to twenty times over the course of the day. Such an undertaking is not only cumbersome and time consuming, but is also potentially very expensive. Transient symptoms of dry eye associated with refractive surgery have been reported to last in some cases from six weeks to six months or more following surgery.

Aside from efforts directed primarily to the alleviation of symptoms associated with dry eye, methods and compositions directed to treatment of the dry eye condition have also been pursued. For example, U.S. Patent No. 5,041,434 (Lubkin) discloses the use of sex steroids, such as conjugated estrogens, to treat dry eye conditions in post-menopausal women; U.S. Patent No. 5,290,572 (MacKeen) discloses the use of finely divided calcium ion compositions to stimulate pre-ocular tear film production; and U.S. Patent No. 4,966,773 (Gressel et al.) discloses the use of microfine particles of one or more retinoids for ocular tissue normalization.

Some recent literature reports suggest that patients suffering from dry eye syndrome disproportionately exhibit the hallmarks of excessive inflammation in relevant ocular tissues, such as the lacrimal and meibomian glands. The use of various compounds to treat dry eye patients, such as steroids [e.g. U.S. Patent No. 5,958,912; Marsh, et al., Topical nonpreserved methylprednisolone therapy for keratoconjunctivitis sicca in Sjogren syndrome, Ophthalmology, 106(4): 811-816 (1999); Pflugfelder, et. al. U.S. Patent No. 6,153,607], cytokine release inhibitors (Yanni, J.M.; et. al. WO 0003705 A1), cyclosporine A [Tauber, J. Adv. Exp. Med. Biol. 1998, 438 (Lacrimal Gland, Tear Film, and Dry Eye Syndromes 2), 969], and 15-HETE (Yanni et. al., US Patent No. 5,696,166), has been disclosed.

Uveitis is an intraocular inflammatory condition that is usually limited to the anterior ocular structures, and can be managed with topical corticosteroids. The inflammatory process can extend behind the lens to affect the pars plana, the vitreous cavity, the choroid, and the retina. These intermediate and posterior manifestations are relatively rare but contribute disproportionately to visual morbidity and present serious therapeutic difficulties. Systemic corticosteroids constitute the first line of treatment for most sight-threatening uveitides. Their long term use is limited by universal and debilitating adverse effects. Second-line agents that allow a reduction in steroid use, such as cyclosporin and azathioprine, offer alternative approaches. Unfortunately their use is frequently limited by a narrow therapeutic window and significant adverse side effects.

Lee *et*. *al*. have disclosed that compounds **1** and **2** inhibit LTB₄-induced chemotaxis of neutrophils as potently as lipoxin A₄ [Lee et. al., Biochemical and Biophysical Research Communications 1991, 180(3), 1416-21]. Lipoxin A₄ and certain analogs thereof have been reported to be antiinflammatory agents (see for example Serhan *et*. *al.,* US patent number 5,441,951). Certain lipoxin analogs have been claimed for treating dry eye (Gamache *et*. *al.,* US Patent No. 6,645,978 B1). However to the best of our knowledge no compounds of the present invention have been described for treating dry eye or uveitis.

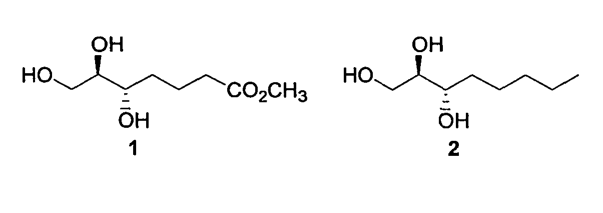

### Summary of the Invention

The present invention is directed to the manufacture of a medicament for the treatment of dry eye and uveitis. According to the uses of the present invention, a 5,6,7-trihydroxyheptanoic acid or analog according to formula (1) is used in the manufactured of a medicament to be administered to a patient. The 5,6,7-trihydroxyheptanoic acid or analog according to formula (1) is preferably to be administered in an ophthalmic composition dosed topically to a patient's eye.

### Detailed Description of the Invention

Unless indicated otherwise, all component amounts are presented on a % (w/v) basis.

The medicament manufactured according to the present invention comprises a compound of formula 1 which is to be administered topically to a mammal in need thereof: 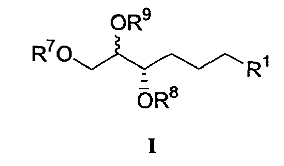 wherein
R¹ is C₂H₅, CO₂R CONR²R³, CH₂OR⁴ or CH₂NR⁵R⁶, where:
R is H, C₁₋₆ straight chain or branched alkyl, C₃₋₆ cycloalkyl, or phenyl;
or R¹ is a carboxylate salt of formula CO₂⁻R⁺, where R⁺ is Li⁺, Na⁺, K⁺, or an ammonium moiety of formula ⁺NR¹⁰R¹¹R¹²R¹³;
R², R³ are independently H, C₁₋₆ alkyl, C₃₋₆ cycloalkyl, benzyl, phenyl, OH, OCH₃, or OC₂H₅, provided that at most only one of R², R³ is OH, OCH₃, or OC₂H₅;
R⁴ is H, C(O)R¹⁴, C₁₋₆ alkyl, C₃₋₆ cycloalkyl, benzyl, or phenyl;
R⁵, R⁶ are independently H, C(O)R¹⁴, C₁₋₆ alkyl, C₃₋₆ cycloalkyl, benzyl, phenyl, OH, OCH₃, or OC₂H₅, provided that at most only one of R², R³ is OH, OCH₃, or OC₂H₅;
R⁷, R³, R⁹ are independently H, CH₃, C₂H₅, C(O)R¹⁴, or CO₂R¹⁵;
or R⁷ and R⁸ or R⁸ and R⁹ together constitute a carbonyl group (C=O), thus forming a cyclic carbonate;
or OR⁸R¹ together form a cyclic ester (a lactone);
R¹⁰-R¹³ are independently H or C₁₋₆ alkyl, each alkyl group optionally bearing an OH or OCH₃ substituent;
R¹⁴ is H, C₁₋₆ alkyl, C₃₋₆ cycloalkyl, benzyl, or phenyl;
R¹⁵ is C₁₋₆ alkyl, C₃₋₆ cycloalkyl, benzyl, or phenyl; and
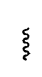 indicates that the OR⁹ substituent can be arranged to afford the R or S absolute configuration:

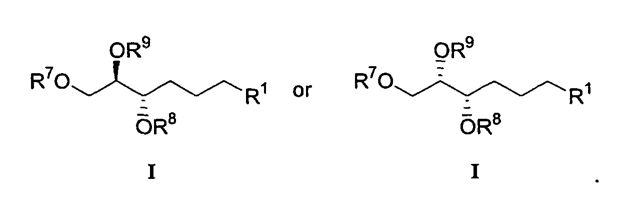

Preferred compounds of formula I are those wherein:
R¹ is C₂H₅, CO₂R or CH₂OR⁴;
R is H, Na⁺, NH₄⁺, CH₃, C₂H₅, n-C₃H₇, or i-C₃H₇;
R⁴ is H, COCH₃, or CH₃; and
R⁷, R⁸, R⁹ are independently H, CH₃, CH₃CO;
or R⁷ and R⁸ or R⁸ and R⁹ together constitute a carbonyl group (C=O), thus forming a cyclic carbonate;
or OR⁸R¹ together form a cyclic ester (a lactone).

Especially preferred are compounds 1 and 2. Compound 1 of formula I is commercially available from Biomol Research Laboratories, Plymouth Meeting, PA. Other compounds of formula I can be prepared as detailed in Lee et. al., Biochemical and Biophysical Research Communications 1991, 180(3), 1416-21.

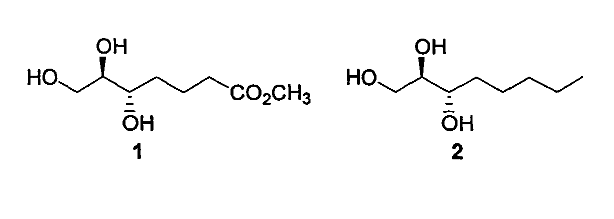

### Example 1: Protective effect of compound 1 in a rabbit model of dry eye.

Compound 1 was evaluated in a rabbit model of dry eye. New Zealand white rabbits (approximately 2.5 kg; obtained from Myrtle's Rabbitry, Thompson Station, TN) were randomized and dosed topically twice a day with either 50µl of compound 1 formulated in 0.064%/BSS® at concentrations of 1, 10, or 100 µM , or with 0.064%/BSS® vehicle. After 24 h the rabbits were anesthetized by subcutaneous administration of ketamine hydrochloride (30 mg/kg) and xylazine (6 mg/kg) and each rabbit received bilateral injections of Conconavilin A (ConA) (300µg/30µl) or saline (30µl). Desiccation was initiated one day following lacrimal gland injection by placing conscious animals in an environmental chamber (20-30% humidity, 75°C). Following 72 hours of exposure to environment, the animals were assessed for corneal staining upon exposure of the cornea to the dye methylene blue; less staining indicates less damage to the cornea. The rabbits were anesthetized by subcutaneous administration of ketamine hydrochloride (30 mg/kg) and xylazine (6 mg/kg). Sutures were placed in each upper and lower eyelid and lifted to form a corneal/conjunctival cup. Methylene blue dye (1 mL, 1% in distilled water) was added to the cup for five minutes and the excess removed by washing with 200 mL of BSS®. The contralateral eye was then stained using the same procedure. Rabbits were euthanized immediately following the staining procedure and the eyes were excised. The corneas were isolated and a 9.5 mm punch of the cornea was placed overnight in 2 mL of acetone/saturated sodiuim sulfate (7:3 v/v). The concentration of the extracted dye was determined spectrophotometrically by measuring its absorbance at λ = 660 nanometers (A660). Percent inhibition was calculated as {1-[(A660_{test item}- A660_{Normal})/(A66_{Bess} - A660_{Normal})]} X 100, where A660_{test item} is the absorbance of dye from ConA-injected eyes dosed with compound 1, A660_{Normal} is the absorbance of dye from saline-injected eyes, and A660_{BSS} is the absorbance of dye in ConA-injected eyes dosed with 0.064% ethanol/BSS® solution vehicle. A higher percent inhibition of staining indicates more protection of the cornea from damage.

A second group of animals was evaluated for tear film quality by measuring each animal's tear breakup time (TBUT). Using the same experimental protocol as above for inducing rabbit ocular damage, TBUT was determined daily by instilling 5 µL of sodium fluorescein into the cul de sac and manually blinking the lids to distribute the fluroescein within the tear film. Under slit lamp observation, the eye was held open and the time whereby one or more black spots or streaks appeared in the precorneal tear film was recorded. The rabbits were euthanized 3 days following ConA injection. Larger TBUT values indicate better tear film quality and more protection from ocular damage. TBUT data is expressed as % of baseline, with baseline TBUT being that observed for saline-injected, vehicle-treafied eyes.

The % inhibition of corneal staining and TBUT data are presented below in table 1, with 15S-HETE (Biomol Research Laboratories, Plymouth Meeting, PA) treatment of ConA-injected eyes used as a positive control.

**Table 1. Effect of Compound 1 on Ocular Damage in Rabbits Induced by Lacrimal Gland ConA Injection Followed by Desiccation**

| Compound | Concentration | % Inhibition of | TBUT, % of |
|---|---|---|---|
| | (µM) | Corneal Staining, | Baseline, ± S.D. |
| | | ± S.D.^{a} | |
| 15S-HETE | 1 | 77 ± 18^{b} | 54 ± 21 ^{b} |
| 1 | 1 | 75 ± 12^{b} | 67 ± 17^{b} |
| 1 | 10 | 54 ± 9^{b,c} | 45 ± 17^{b} |
| 1 | 100 | 38 ± 34 ^{b,c} | 51 ± 18^{b} |

| | | | |
|---|---|---|---|
| ^{a}S.D. = Standard Deviation. ^{b}p<0.01 (Dunnett's t-test) compared to vehicle. ^{c}p<0.01 (Dunnett's t-test) compared to 15S-HETE. | | | |

### Example 2: Protective effect of compound 1 in a rat model of uveitis.

Compound 1 was evaluated for its ability to suppress neutrophil influx into the rat eye in a model of endotoxin-induced uveitis. The compound was prepared at concentrations of 0.01%, 0.1%, 1.0% w/v in an ophthalmic suspension vehicle, and dexamethasone (Sigma-Aldrich Company, St. Louis, MO) formulated in the same vehicle served as reference compound. Uveitis was induced by subplantar injection of endotoxin (200 µg in 0.1 mL saline) in the right hind paw of female Lewis rats (5/group). Test compound of vehicle (5 µL) was administered topically to each eye of the experimental animals at the time of endoxtoxin injection and again 4 hours later. Twenty-four hours post endotoxin injection, animals were sacrificed by CO₂ inhalation, and total ocular neutrophil (PMN) content was assesed indirectly by determination of myeloperoxidase activity. Ocular PMN content in each group was then compared with that observed in the vehicle-treated group using Dunnet's t-test. The results are shown below in table 2.

**Table 2. Effect of Compound 1 on Endotoxin-Induced Uveitis in Rats Following Topical Ocular Administration**

| Compound | Concentration | Myeloperoxidase | % inhibition |
|---|---|---|---|
| | (%, w/v) | (µM/min/100 mg) | |
| | | (x ± standard | |
| | | deviation) | |
| Carbopol Vehicle | -- | 164 ± 46 | -- |
| 1 | 0.01 | 143 ± 64 | 13 |
| | 0.1 | 161 ± 37 | 2 |
| | 1.0 | 97 ± 35 | 41 * |
| Dexamethasone | 0.1 | 15 ± 7 | 91 * |

| | | | |
|---|---|---|---|
| *p<0.01, Dunnett's t-test. | | | |

According to the present invention, the medicament comprising a compound of formula I is to be administered in a pharmaceutically acceptable carrier for topical ophthalmic administration. The compositions are formulated in accordance with methods known in the art. The compositions may contain more than one compound of formula 1. Additionally, the compositions may contain a second drug, other than a compound of formula 1.

The compositions of the present invention contain a pharmaceutically effective amount of a compound of formula 1. As used herein, "a pharmaceutically effective amount" means an amount sufficient to reduce or eliminate uveitis or dry eye symptoms. Generally, the compositions of the present invention will contain from 0.00001 to 0.01% of a compound of formula I for treating dry eye, and from 0.01% to 3% of a compound of formula 1 for treating uveitis. Preferably, the compositions of the present invention will contain from 0.00003 to 0.001% of a compound of formula I for treating dry eye, and from 0.1% to 1% of a compound of formula 1 for treating uveitis.

The compositions administered according to the present invention may also include various other ingredients, including surfactants, tonicity agents, buffers, preservatives, co-solvents and viscosity building agents.

Various tonicity agents may be employed to adjust the tonicity of the composition, preferably to that of natural tears for ophthalmic compositions. For example, sodium chloride, potassium chloride, magnesium chloride, calcium chloride, dextrose and/or mannitol may be added to the composition to approximate physiological tonicity. Such an amount of tonicity agent will vary, depending on the particular agent to be added. In general, however, the compositions will have a tonicity agent in an amount sufficient to cause the final composition to have an ophthalmically acceptable osmolality (generally about 150 - 450 mOsm, preferably 250 - 350 mOsm).

An appropriate buffer system (e.g., sodium phosphate, sodium acetate, sodium citrate, sodium borate or boric acid) may be added to the compositions to prevent pH drift under storage conditions. The particular concentration will vary, depending on the agent employed. Preferably, however, the buffer will be chosen to maintain a target pH within the range of pH 5.5 - 8.

Other compounds designed to lubricate, "wet," approximate the consistency of endogenous tears, aid in natural tear build-up, or otherwise provide temporary relief of dry eye symptoms and conditions upon ocular administration to the eye are known in the art and may be included in the compositions of the present invention. Such compounds may enhance the viscosity of the composition, and include: monomeric polyols, such as, glycerol, propylene glycol, ethylene glycol; polymeric polyols, such as, polyethylene glycol, hydroxypropylmethyl cellulose ("HPMC"), carboxy methylcellulose sodium, hydroxy propylcellulose ("HPC"), dextrans, such as, dextran 70; water soluble proteins, such as gelatin; and vinyl polymers, such as, polyvinyl alcohol, polyvinylpyrrolidone, povidone and carbomers, such as, carbomer 934P, carbomer 941, carbomer 940, carbomer 974P.

Topical ophthalmic products are typically packaged in multidose form. Preservatives are typically required to prevent microbial contamination during use. Suitable preservatives include: benzalkonium chloride, chlorobutanol, benzododecinium bromide, methyl paraben, propyl paraben, phenylethyl alcohol, edetate disodium, sorbic acid, polyquaternium-1, or other agents known to those skilled in the art. Such preservatives are typically employed at a level of from 0.001 to 1.0% w/v. Unit dose compositions of the present invention will be sterile, but typically will not contain a preservative and will be unpreserved.

Generally, 1-2 drops of such compositions will be administered from once to many times per day.

Representative eye drop formulations are provided below in Example 3 for treating dry eye and in Example 4 for treating uveitis.

### Example 3

| Ingredient | Concentration (%w/v) |
|---|---|
| Compound of formula 1 | 0.00003-0.001 |
| Ethanol | 0.03 - 0.2 |
| Boric Acid | 0.1 - 0.3 |
| Polyoxyl 40 Stearate | 0.1 |
| Edetate Disodium | 0.01 |
| Polyquaternium 1 | 0.001 |
| NaOH/HCl | q.s. to pH 6-8 |
| Purified Water | q.s. to 100% |

### Example 4

| Ingredient | Concentration (%w/v) |
|---|---|
| Compound of formula 1 | 0.1-1.0 |
| Hydroxypropyl methylcellulose | 0.1 - 0.5 |
| Dextran 70 | 0.1 |
| Sodium Chloride | 0.8 |
| Potassium Chloride | 0.12 |
| Dibasic Sodium Phosphate | 0.025 |
| Edetate Disodium | 0.01 |
| Polyquaternium-1 | 0.001 - 0.005 |
| NaOH/HCl | q.s. to pH 6 - 8 |
| Purified Water | q.s. to 100 |

This invention has been described by reference to certain preferred embodiments. The embodiments described above are therefore considered to be illustrative in all respects and not restrictive, the scope of the invention being indicated by the appended claims rather than by the foregoing description.

## Claims

1. Use of a pharmaceutically acceptable carrier and a pharmaceutically effective amount of a compound of formula 1: 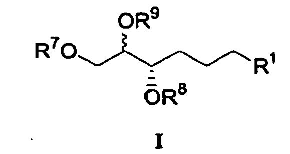 wherein
R¹ is C₂H₅, CO₂R, CONR²R³, CH₂OR⁴, or CH₂NR⁵R , where:
R is H, C₁₋₆ straight chain or branched alkyl, C₃₋₈ cycloalkyl, or phenyl; or R¹ is a carboxylate salt of formula CO₂ ⁻R⁺, where R⁺ is Li⁺, Na⁺, K⁺,
or an ammonium moiety of formula ⁺NR¹⁰R11R¹²R¹³;
R², R³ are independently H, C₁₋₆ alkyl, C₃₋₆ cycloalkyl, benzyl, phenyl, OH, OCH₃, or OC₂H₅, provided that at most only one of R², R³ is OH, OCH₃, or OC₂H₅;
R⁴ is H, C(O)R¹⁴, C₁₋₆ alkyl, C₃₋₆ cycloalkyl, benzyl, or phenyl;
R⁵, R⁶ are independently H, C(O)R¹⁴, C₁₋₆ alkyl, C₃₋₆ cycloalkyl, benzyl, phenyl, OH, OCH₃, or OC₂H₅, provided that at most only one of R², R³ is OH, OCH₃, or OC₂H₅;
R⁷, R⁸, R⁹ are independently H, CH₃, C₂H₅, C(O)R¹⁴, or CO₂R¹⁵;
or R⁷ and R⁸ or R⁸ and R⁹ together constitute a carbonyl group;
or OR⁸R¹ together form a cyclic ester;
R¹⁰, R¹¹, R¹² , R¹³ are independently H or C₁₋₆ alkyl, each alkyl group optionally bearing an OH or OCH₃ substituent;
R¹⁴ is H, C₁₋₆ alkyl, C₃₋₆ cycloalkyl, benzyl, or phenyl; and
R¹⁵ is C₁₋₆ alkyl, C₃₋₆ cycloalkyl, benzyl, or phenyl
for the preparation of a medicament for the treatment of dry eye or uveitis in a mammal wherein the medicament is to be administered topically to the eye.

2. Use of Claim 1 wherein for the compound of formula 1:
R¹ is C₂H₅, CO₂R, or CH₂OR⁴;
R is H, Na⁺, NH₄⁺, CH₃, C₂H₅, n-C₃H₇, or i-C₃H₇;
R⁴ is H, COCH₃, or CH₃; and
R⁷, R⁸, R⁹ are independently H, CH₃, CH₃CO;
or R⁷ and R⁸ or R⁸ and R⁹ together constitute a carbonyl group;
or OR⁸R¹ together form a cyclic ester.

3. Use of Claim 2 wherein the compound of formula 1 has the configuration:

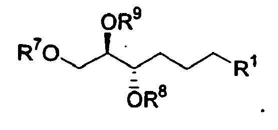

4. Use of Claim 1 wherein the compound of formula 1 has the configuration:

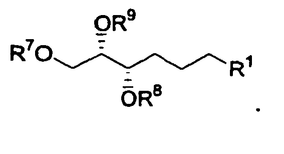

5. Use of claim 3, wherein the medicament is for treating uveitis.

6. Use of claim 3, wherein the medicament is for treating dry eye.

7. Use of claim 5, wherein the compound is selected from the group consisting of:

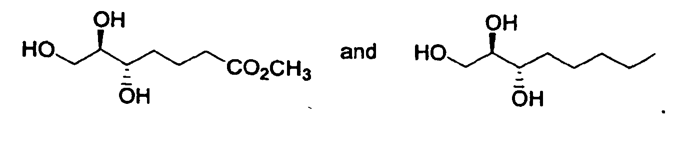

8. Use of claim 6, wherein the compound is selected from the group consisting of:

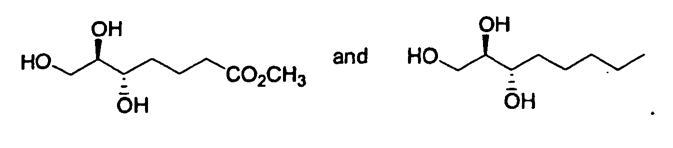

9. Use of Claim 5, wherein the pharmaceutically effective amount is from 0.1 to 1% (w/v).

10. Use of Claim 6 wherein the pharmaceutically effective amount is from 0.00003 to 0.001% (w/v).

11. Use of Claim 9, wherein the pharmaceutically acceptable carrier comprises one or more ingredients selected from the group consisting of surfactants; tonicity agents; buffers; preservatives; co-solvents; and viscosity building agents.

12. Use of claim 10, wherein the pharmaceutically acceptable carrier comprises one or more ingredients selected from the group consisting of surfactants; tonicity agents; buffers; preservatives; co-solvents; and viscosity building agents.

## Patentansprüche

1. Verwendung eines pharmazeutisch akzeptablen Trägers und einer pharmazeutisch akzeptablen Menge einer Verbindung der Formel I: 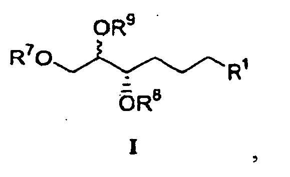 worin
R¹ C₂H₅, CO₂R, CONR²R³, CH₂OR₄ oder CH₂NR⁵R⁶ ist, wobei:
R H, geradkettiges oder verzweigtes C₁₋₆-Alkyl, C₃₋₆-Cycloalkyl oder Phenyl ist;
oder R¹ ein Carboxylatsalz der Formel CO₂-R⁺ ist, wobei R⁺ Li⁺, Na⁺, K⁺ oder eine Ammoniumkomponente der Formel +NR¹⁰R¹¹R¹²R¹³ ist;
R², R³ unabhängig voneinander H, C₁₋₆-Alkyl, C₃₋₆-Cycloalkyl, Benzyl, Phenyl, OH, OCH₃ oder OC₂H₅ sind, vorausgesetzt, dass höchstens einer von R², R³ OH, OCH₃ oder OC₂H₅ ist; R⁴ H, C(O)R¹⁴, C₁₋₆-Alkyl, C₃₋₆-Cycloalkyl, Benzyl oder Phenyl ist;
R⁵, R⁶ unabhängig voneinander H, C(O)R¹⁴, C₁₋₆-Alkyl, C₃₋₆-Cycloalkyl, Benzyl, Phenyl, OH, OCH₃ oder OC₂H₅ sind, vorausgesetzt, dass höchstens einer von R², R³ OH, OCH₃ oder OC₂H₅ ist;
R⁷, R⁸, R⁹ unabhängig voneinander H, CH₃, C₂H₅, C(O)R¹⁴ oder CO₂R¹⁵ sind;
oder R⁷ und R⁸ oder R⁸ und R⁹ zusammen eine Carbonylgruppe bilden;
oder OR⁸R¹ zusammen einen cyclischen Ester bilden;
R¹⁰, R¹¹, R¹² , R¹³ unabhängig voneinander H oder C₁₋₆-Alkyl sind, wobei jede Alkylgruppe gegebenenfalls einen OH- oder OCH₃-Substituenten trägt;
R¹⁴ H, C₁₋₆-Alkyl, C₃₋₆-Cycloalkyl, Benzyl oder Phenyl ist und
R¹⁵ C₁₋₆-Alkyl, C₃₋₆-Cycloalkyl, Benzyl oder Phenyl ist,
zur Herstellung eines Medikaments für die Behandlung von trockenen Augen oder Uveitis bei einem Säuger, wobei das Medikament topisch an das Auge verabreicht werden soll.

2. Verwendung nach Anspruch 1, wobei für die Verbindung der Formel I:
R¹ C₂H₅, CO₂R oder CH₂OR⁴ ist;
R H, Na⁺, NH₄⁺, CH₃, C₂H₅, n-C₃H₇ oder i-C₃H₇ ist;
R⁴ H, COCH₃ oder CH₃ ist und
R⁷, R⁸, R⁹ unabhängig voneinander H, CH₃, CH₃CO sind;
oder R⁷ und R⁸ oder R⁸ und R⁹ zusammen eine Carbonylgruppe bilden;
oder OR⁸R¹ zusammen einen cyclischen Ester bilden.

3. Verwendung nach Anspruch 2, wobei die Verbindung der Formel I die Konfiguration: 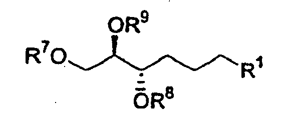 hat.

4. Verwendung nach Anspruch 1, wobei die Verbindung der Formel I die Konfiguration: 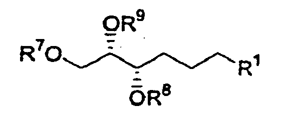 hat.

5. Verwendung nach Anspruch 3, wobei das Medikament zur Behandlung von Uveitis dient.

6. Verwendung nach Anspruch 3, wobei das Medikament zur Behandlung trockener Augen dient.

7. Verwendung nach Anspruch 5, wobei die Verbindung aus der Gruppe ausgewählt ist, bestehend aus:

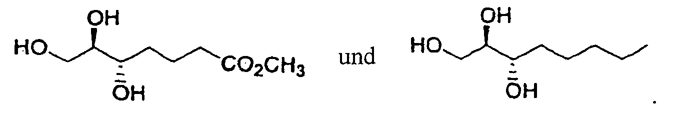

8. Verwendung nach Anspruch 6, wobei die Verbindung aus der Gruppe ausgewählt ist, bestehend aus:

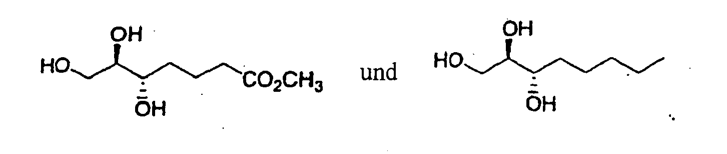

9. Verwendung nach Anspruch 5, wobei die pharmazeutisch wirksame Menge 0,1 bis 1 % (Gew./Vol.) beträgt.

10. Verwendung nach Anspruch 6, wobei die pharmazeutisch wirksame Menge 0,00003 bis 0,001 % (Gew./Vol.) beträgt.

11. Verwendung nach Anspruch 9, wobei der pharmazeutisch akzeptable Träger einen oder mehrere Inhaltsstoffe, ausgewählt aus der Gruppe, bestehend aus oberflächenaktiven Mitteln; Tonizitätsmitteln; Puffern; Konservierungsmitteln; Hilfslösungsmitteln und Viskositätsaufbaumitteln, umfasst.

12. Verwendung nach Anspruch 10, wobei der pharmazeutisch akzeptable Träger einen oder mehrere Inhaltsstoffe, ausgewählt aus der Gruppe, bestehend aus oberflächenaktiven Mitteln; Tonizitätsmitteln; Puffern; Konservierungsmitteln; Hilfslösungsmitteln und Viskositätsaufbaumitteln, umfasst.

## Revendications

1. Utilisation d'un excipient pharmaceutiquement acceptable et d'une quantité pharmaceutiquement efficace d'un composé de formule 1 : 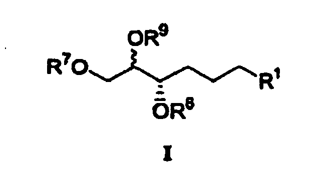 où
R¹ est un C₂H₅, CO₂R, CONR²R³, CH₂OR⁴ ou CH₂NR⁵R⁶, où :
R est un H, un alkyle à chaîne droite ou ramifié, un cycloalkyle en C₃₋₆, ou un phényle,
ou R¹ est un sel de carboxylate de formule CO₂⁻R⁺,
où R⁺ est un Li⁺, Na⁺, K⁺, ou une portion d'ammonium de formule +NR¹⁰R¹¹R¹²R¹³ ;
R², R³ sont indépendamment un H, un alkyle en C₁₋₆, un cycloalkyle en C₃₋₆, un benzyle, un phényle, un OH, un OCH₃, ou un OC₂H₅, à condition qu'au plus uniquement un élément parmi R², R³ soit un OH, OCH₃ ou OC₂H₅ ;
R⁴ est un H, C(O)R¹⁴, un alkyle en C₁₋₆, un cycloalkyle en C₃₋₆, un benzyle ou un phényle ;
R⁵, R⁶ sont indépendamment un H, un C(O)R¹⁴, un alkyle en C₁₋₆, un cycloalkyle en C₃₋₆, un benzyle, un phényle, un OH, un OCH₃, ou un OC₂H₅, à condition qu'au plus uniquement un élément parmi R², R³ soit un OH, OCH₃ ou OC₂H₅
R⁷, R⁸, R⁹ sont indépendamment un H, CH₃, C₂H₅, C(O)R¹⁴ ou CO₂R¹⁵;
ou R⁷ et R⁸ ou R⁸ et R⁹ constituent ensemble un groupe carbonyle, ou OR⁸R¹ forment ensemble un ester cyclique ;
R¹⁰, R¹¹, R¹², R¹³ sont indépendamment un H ou alkyle en C₁₋₆, chaque groupe alkyle portant éventuellement un substituant OH ou OCH₃ ;
R¹⁴ est un H, un alkyle en C₃₋₆, un benzyle ou un phényle, et
R¹⁵ est un alkyle en C₁₋₆, un cycloalkyle en C₃₋₆, un benzyle ou un phényle
pour la préparation d'un médicament pour le traitement des yeux secs ou de l'uvéite chez un mammifère, dans lequel le médicament doit être administré par voie topique dans l'oeil.

2. Utilisation selon la revendication 1 dans laquelle, pour le composé de formule I :
R¹ est un C₂H₅, CO₂R, ou CH₂OR⁴ ;
R est un H, Na⁺, NH₄⁺, CH₃, C₂H₅, n-C₃H₇, ou i-C₃H₇ ;
R⁴ est un H, un COCH₃ ou CH₃ ; et
R⁷, R⁸, R⁹ sont indépendamment un H, CH₃, CH₃CO;
ou R⁷ et R⁸ ou R⁸ et R⁹ constituent ensemble un groupe carbonyle ;
ou OR⁸R¹ forment ensemble un ester cyclique.

3. Utilisation selon la revendication 2, dans laquelle le composé de formule I a la configuration :

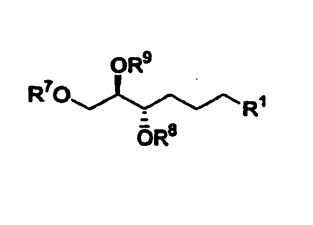

4. Utilisation selon la revendication 1, dans laquelle le composé de formule I a la configuration :

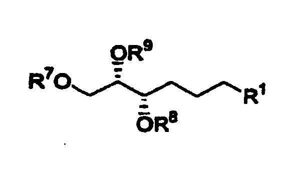

5. Utilisation selon la revendication 3, dans laquelle le médicament est destiné à traiter l'uvéite.

6. Utilisation selon la revendication 3, dans laquelle le médicament est destiné à traiter les yeux secs.

7. Utilisation selon la revendication 5, dans laquelle le composé est choisi dans le groupe constitué de :

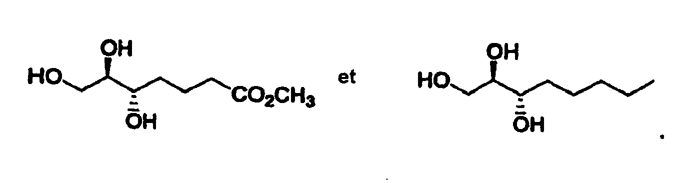

8. Utilisation selon la revendication 6, dans laquelle le composé est choisi dans le groupe constitué de :

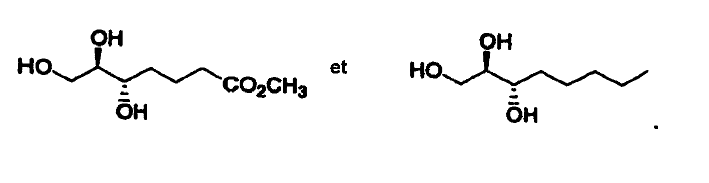

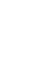

9. Utilisation selon la revendication 5, dans laquelle la quantité pharmaceutiquement efficace est comprise entre 0,1 et 1% en poids

10. Utilisation selon la revendication 6, dans laquelle la quantité pharmaceutiquement efficace est comprise entre 0,00003 et 0,001% (en poids).

11. Utilisation selon la revendication 9, dans laquelle l'excipient pharmaceutiquement acceptable comprend un ou plusieurs ingrédients choisis dans le groupe constitué de tensioactifs ; d'agents de toxicité, de tampons, de conservateurs, de co-solvants et d'agents formateurs de viscosité.

12. Utilisation selon la revendication 10, dans laquelle l'excipient pharmaceutiquement acceptable comprend un ou plusieurs ingrédients choisis dans le groupe constitué de tensioactifs ; d'agents de tonicité, de tampons, de conservateurs, de co-solvants, et d'agents de formation de viscosité.
